Europäisches Patentamt

European Patent Office

Office européen des brevets

(19)

(11) Veröffentlichungsnummer: **0 284 545**
**A1**

(12) **EUROPÄISCHE PATENTANMELDUNG**

(21) Anmeldenummer: **88730012.7**

(22) Anmeldetag: **16.01.88**

(51) Int. Cl.⁴: **A 61 F 2/28**

(30) Priorität: **26.02.87 DE 8703120**

(43) Veröffentlichungstag der Anmeldung:
**28.09.88 Patentblatt 88/39**

(84) Benannte Vertragsstaaten:
**CH DE ES FR GB IT LI**

(71) Anmelder: **MECRON medizinische Produkte GmbH**
**Nunsdorfer Ring 23-29**
**D-1000 Berlin 48 (DE)**

(72) Erfinder: **Kranz, Curt, Dipl.-Ing.**
**Landshuter Str. 5**
**D-1000 Berlin 30 (DE)**

**Ahrens, Uwe, Dipl.-Ing.**
**Nürnberger Str. 46**
**D-1000 Berlin 30 (DE)**

**Marquardt, Ernst, Prof. Dr. med.**
**Schlierbacher Landstrasse 200a**
**D-8900 Heidelberg (DE)**

**Philipp, Alexander**
**Leinstr. 33**
**D-3000 Hannover (DE)**

(74) Vertreter: **Christiansen, Henning, Dipl.-Ing.**
**CHRISTIANSEN & NINNEMANN Dietrich-Schäfer-Weg 21**
**D-1000 Berlin 41 (DE)**

(54) **Stumpfkappe.**

(57) Stumpfkappe, enthaltend einen Auflagekörper (1) für den Knochenstumpf, insbesondere für den Femurstumpf, der mit der Kopffläche am unteren Abschluß des Knochenstumpfes anliegt, wobei der Auflagekörper (1) im wesentlichen zylindrisch ausgebildet ist und eine in wesentlichen koaxiale stirnseitige Ausnehmung (3) mit Innengewinde (4, 5) aufweist.

Fig. 1

EP 0 284 545 A1

**Beschreibung**

Stumpfkappe

Die Erfindung betrifft eine Stumpfkappe der im Oberbegriff des Anspruchs 1 angegebenen Art.

Eine derartige Kappe ist aus der FR-PS 10 46 920 bekannt. Dabei wird in den Femur-Knochenschaft ein Implantat ein geführt, an dessen unterem Ende ein pilzförmig ausgebildeter Fuß angeordnet ist, der mit seiner Kopffläche am unteren Abschluß des Knochenstumpfes anliegt. Der Durchmesser des am Knochenstumpf anliegenden Endes ist dabei nur halb so groß wie der Durchmesser des Knochenstumpfes, so daß das Implantat keine Entlastung des Tubers bringt.

Bekanntlich besitzen Prothesen an ihrem oberen Ende einen Prothesenschaft, in dem sich das Stumpfende des Beines befindet. Dabei stützt sich bei Belastung der sogenannte Tuber auf dem oberen Rand des Prothesenschaftes ab, während der Beinstumpf in dem Porthesenschaft belastungsfrei bleibt. Der Tuber ist aber an sich kein Stützknochen und zur Aufnahme von Belastungen nicht geeignet. Wegen dieser Abstützung des Tubers auf dem Prothesenschaft treten daher bei Prothesenträgern häufig unangenehme Schmerzen auf. Personen mit einer Prothese scheuen sich somit meistens, die Prothese zu belasten, indem das Körpergewicht möglichst auf das andere gesunde Bein verlagert wird.

Die aus der DE-PS 31 25 268 bekannte Stumpfkappe weist zwar eine breitere Auflage auf, jedoch besteht die Gefahr, daß durch den auch bei dieser Auflage vorhandenen Fuß, der in den Markraum eingebracht wird, wegen der damit verbundenen Knochmarkentfernung später ein Absterben von Gewebe verursacht wird.

Der im Anspruch 1 angegebenen Erfindung liegt die Aufgabe zugrunde, ein Implantat zu schaffen, das die Übertragung der bei Benutzung einer Prothese auftretenden Belastung über die Gewebeschicht zwischen dem unteren Ende, ins besondere des Beinstockes und dessen Fußfläche, unmittelbar auf den Knochenstumpf bei langfristiger Erhaltung des verbleibenden Stumpfs ermöglicht.

Durch das erfindungsgemäße Implantat wird die Möglichkeit geschaffen, daß die Prothese auf dem Beinstumpf bzw. dem darin befindlichen Femur-Knochenstumpf - also dem in Beinstumpf verbliebenen Oberschenkelknochen - ohne Zuhilfenahme eines Fußes befestigt wird. Die Anwendung kann entsprechend auch für andere Gliedmaßen erfolgen.

Besonders vorteilhaft bei der Erfindung ist, daß das Befestigen des Aufsatzes mit einer einzigen Handhabung ohne Bohrungen und dergleichen erfolgen kann. Ein Aufschrauben erfolgt bevorzugt durch Drehmomentübertragung mittels eines Werkzeugs in Wechselwirkung mit Ausnehmungen, die in der Außenkontour vorgesehen sind.

Die Vorteile der erfindungsgemäßen Stumpfkappen liegen weiterhin in der Vermeidung von Spitzenwachstum und der Durchsprießungsgefahr bzw. der dabei erforderlich werdenden Nachamputation. Zum anderen wird das geordnete Knochenwachstum beim Kind durch die Belastung der Epiphyse stimuliert. Ein weiterer Vorteil besteht in der teilweisen Belastung des Stumpfendes bei einer Amputation und einer starken Entlastung des Tuber ossis ischii im Falle der Anwendung beim Femur. Hervorzuheben ist auch die bessere Kraftverteilung im Gewebe. In der zusätzlichen Befestigungsmöglichkeit des Muskelgewebes am Implantat unter gleichzeitiger Vermeidung der sonst üblichen Bohrkanäle der Myodese liegt ein weiterer Vorteil der erfindungsge mäßen Stumpfkappen. Neben einer ungestörten bzw. wenig gestörten Knochenversorgung wird auch die Erhaltung der Durchblutung am Stumpfende gesichert und der Markraum verschlossen. Ein weiterer positiver Aspekt liegt in der einfachen Befestigung der erfindungsgemäßen Stumpfkappe durch einen Schraubvorgang. Die Verriegelung erfolgt somit sowohl mechanisch als auch biologisch.

Vortielhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

Figur 1 ein Ausführungsbeispiel des Auflagekörpers der erfindungsgemäßen Stumpfkappe in axialer Schnittdarstellung,

Figur 2 der Auflagekörper gemäß Figur 1 in der Draufsicht,

Figur 3 eine Abdeckkappe für den Auflagekörper in radialer Schnittdarstellung und

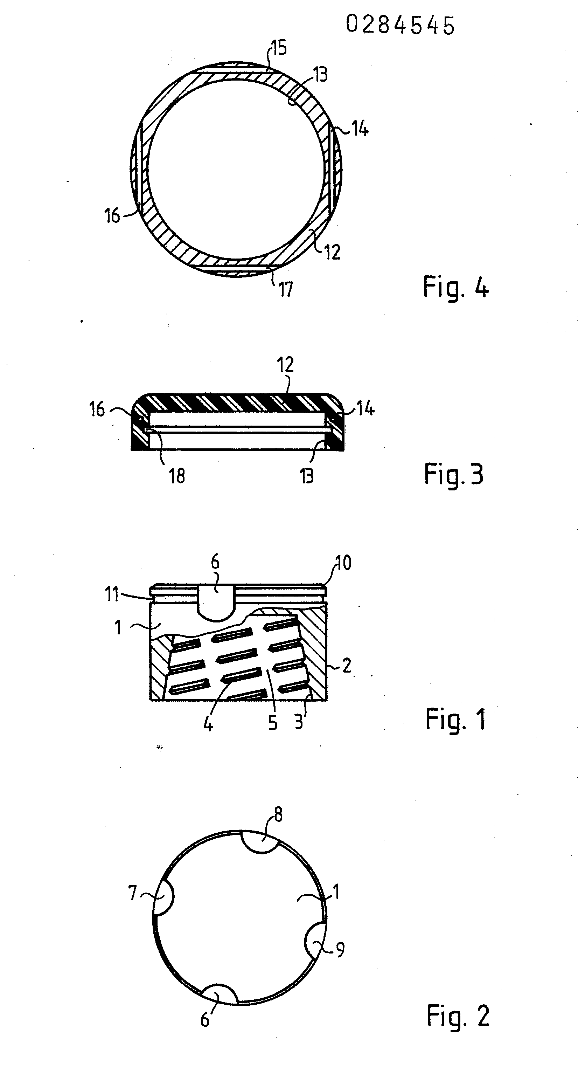

Figur 3 die Abdeckkappe gemäß Figur 2 in axial geschnittener Darstellung.

Bei dem in Figur 1 dargestellten Auflagekörper 1, der eine im wesentlichen zylindrische Außenwandung 2 aufweist, ist im Inneren eine konische koaxiale Ausnehmung 3 vorgesehen, die mit einem Innengewinde versehen ist. Die Öffnung der Ausnehmung befindet sich auf der der geschlossenen Deckfläche gegenüberliegenden Seite. Das Innengewinde ist ein konisches, sich zum Inneren der Kappe hin verjüngendes selbstschneidendes Gewinde mit dem Profil eines Knochenschraubengewindes, wobei nach innen gerichtet Tragflanken 4 vorgesehen sind, welche von Schneidnuten 5 unterbrochen sind. Auf diese Weise kann der Auflagekörper in den meisten Fällen auf den Knochenstumpf direkt aufgeschraubt werden. Im übrigen kann eine Nachbearbeitung mit einem entsprechenden Fräser oder einer Raspel vorgenommen werden.

Der Konus ist der Verjüngung des jeweiligen Knochentyps angepaßt, für den die Anwendung vorgesehen ist, so daß nach entsprechender Auswahl einer Kappenausführung mit geeignetem Innendurchmesser diese direkt aufschraubbar ist.

Bei nicht dargestellten weiteren Varianten des Ausführungsbeispiels kann durch eine leicht (konkav) ballige Gestaltung der das selbstschneidende Innengewinde tragenden Innenfläche der Ausnehmung ein Aufschrauben in auch nachträglich angepaßter Richtung erfolgen. Die genaue Ausrichtung des mit einem selbstschneidenden Gewinde versehehen Auflagekörpers wird dann nach der eventuellen Nachbearbeitung des Knochenstumpfes vorgenommen. Bei konischem Innengewinde liegt hingegen die Schraubrichtung fest und wird durch die Bearbeitung des Knochenstumpfes definiert.

In Figur 2 ist erkennbar, daß der Auflagekörper in seinem vom Knochen abgewandten Bereich Ausnehmungen 6 bis 9 in der Nähe der äußeren Kante aufweist, in die ein Schraubwerkzeug zwecks Übertragung eines Ein- bzw. Ausschraub-Drehmoments eingreifen kann.

Unterhalb einer Fase 10 ist im zylindermantelförmigen Bereich der Außenoberfläche ein Einstich 11 vorgesehen, der einer Halt für die in den Figuren 3 und 4 dargestellt Kappe 12 bildet. Diese Kappe weist in ihrem Inneren eine Ausnehmung 13 auf, die geringfügig kleiner dimensioniert ist als die Auflage 1, so daß sie auf diese aufschnappbar ist, wenn sie aus einem elastischen Werkstoff besteht. Die Kappe 12 ist aus Polyethylen gefertigt, wobei hier sehnenförmig laufende Bohrungen 14 bis 17 im Randbereich vorgesehen sind, durch die Fäden hindurchgezogen werden könnnen, so daß die Kappe mit chirurgischem Nahtmaterial festgenäht werden kann. Die Kappe 12 weist ihrerseits an der Innenseite ebenfalls einen umlaufenden Einstich 18 auf, der bei elastischer Ausführung der Kappe die zylindrischen Innenwandungen in zwei Bereiche unterteilt, die somit jeweils ein eigenes Dichtungssystem bilden. Die Kappe 12 deckt die Ausnehmungen 6 bis 9 vollkommen ab, so daß die Kappe mit Aufsatz nach außen hin einen vollständig geschlossenen Flächenbereich darbietet.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.

## Patentansprüche

1. Stumpfkappe, enthaltend einen Auflagekörper für den Knochenstumpf, insbesondere für den Femurstumpf, der mit der Kopffläche am unteren Abschluß des Knochenstumpfes anliegt,
**dadurch gekennzeichnet,**
daß der Auflagekörper (1)
im wesentlichen zylindrisch ausgebildet ist und
eine im wesentlichen koaxiale stirnseitige Ausnehmung (3) mit Innengewinde (4, 5) aufweist.

2. Stumpfkappe nach Anspruch 1, **dadurch gekennzeichnet,** daß das Gewinde (4, 5) selbstschneidend ausgebildet ist.

3. Stumpfkappe nach einem der vorangehenden Ansprüche, **dadurch gekennzeichnet,** daß das Gewinde (4, 5) konisch verjüngend ausgebildet ist.

4. Stumpfkappe nach einem der vorangehenden Ansprüche, **dadurch gekennzeichnet,** daß das Gewinde (4, 5) ein Normgewinde für Knochenschrauben bildet.

5. Stumpfkappe nach einem der vorangehenden Ansprüche, **dadurch gekennzeichnet,** daß mindestens eine von der dem Knochen abgewandten Seite erreichbare Ausnehmung (6 bis 9) vorgesehen sind, welche einen Drehmomentanschluß für ein Schraubwerkzeug bilden.

6. Stumpfkappe nach einem der vorangehenden Ansprüche, **dadurch gekennzeichnet,** daß die dem Knochen abgewandte Deckfläche eine abgeschrägte Kante (10) aufweist.

7. Stumpfkappe nach einem der vorangehenden Ansprüche, **dadurch gekennzeichnet,** daß in der Nähe der dem Knochen abgewandten Deckfläche ein umlaufender Einstich (11) vorgesehen ist.

8. Stumpfkappe nach einem der vorangehenden Ansprüche, **dadurch gekennzeichnet,** daß der Auflagekörper aus Polyethylen oder Metall besteht.

9. Stumpfkappe nach einem der vorangehenden Ansprüche, **dadurch gekennzeichnet,** daß der Auflagekörper eine flexible Kappe (12) aufweist, die einen den Mantel teilweise übergreifenden Rand enthält.

10. Stumpfkappe nach den Ansprüchen 7 und 9, **dadurch gekennzeichnet,** daß der Rand eine in den Einstich eingreifende Sicke aufweist.

11. Stumpfkappe nach einem der Ansprüche 9 oder 10, **dadurch gekennzeichnet,** daß die Kappe aus Polyethylen besteht.

0 2 8 4 5 4 5

Fig. 4

Fig. 3

Fig. 1

Fig. 2

Europäisches
Patentamt

**EUROPÄISCHER RECHERCHENBERICHT**

Nummer der Anmeldung

EP 88 73 0012

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y,D | FR-A-1 046 920 (PIGAGLIO) <br> * Insgesamt * <br> --- | 1 | A 61 F 2/28 |
| Y | US-A-3 053 251 (BLACK) <br> * Spalte 3, Zeilen 62-74; Figur 5 * <br> --- | 1 | |
| A | US-A-4 005 495 (LOCKE) <br> * Spalte 3, Zeile 30 - Spalte 4, Zeile 13; Figuren 1-3 * <br> --- | 1,3 | |
| A | EP-A-0 017 930 (FELDMÜHLE) <br> * Seite 12, Zeilen 14-21; Figuren 4,6-9 * <br> --- | 1,3 | |
| A,P | DE-U-8 631 192 (MECRON) <br> * Seiten 8,9; Ansprüche 5-11; Figuren 2,3,6,7 * <br> --- | 1,5-11 | |
| A | EP-A-0 006 408 (HOWMEDICA) <br> * Seite 4, Zeile 9 - Seite 5, Zeile 12; Figuren * <br> --- | 2,8 | RECHERCHIERTE SACHGEBIETE (Int. Cl.4) |
| A | DE-A-3 439 993 (FRIEDRICHSFELD) <br> * Seite 3, Abschnitt 3; Seite 4, Abschnitt 2; Figuren * <br> --- | 2,3,5,8 | A 61 F |
| A | DE-A-2 548 077 (FELDMÜHLE) <br> --- | | |
| A | US-A-4 547 912 (SHERVA-PARKER) <br> --- | | |
| A,D | DE-A-3 125 268 (NICOLAI) <br> ----- | | |

Der vorliegende Recherchenbericht wurde für alle Patentansprüche erstellt

| Recherchenort | Abschlußdatum der Recherche | Prüfer |
|---|---|---|
| DEN HAAG | 31-05-1988 | KLEIN C. |